# FASCICULE DE BREVET EUROPEEN

(11) **EP 0 846 721 B1**
(45) Date de publication et mention de la délivrance du brevet: **05.06.2002**
(21) Numéro de dépôt: 97402686.6
(22) Date de dépôt: 10.11.1997
(51) Int. Cl.: C08K 5/3417, A61K 7/42

(54) **Utilisation de mélatonine dans une composition pour stabiliser les polyméres gélifiants hydrophiles**
Verwendung von Melatonin in einer Zusammensetzung zum Stabilisieren gelbildender hydrophiler Polymere
Use of melatonin in a composition for stabilising gelable hydrophilic polymers

(30) Priorité: 06.12.1996 FR 9615045
(43) Date de publication de la demande: 10.06.1998
(73) Titulaire: L'OREAL, 75008 Paris (FR)
(72) Inventeur: Louvet, Nathalie, 94550 Chevilly Larue (FR); Lebreton, Franqoise, 91440 Bures/S/Yvette (FR)
(74) Mandataire: Tezier Herman, Béatrice

(56) Documents cités:
- EP-A- 0 438 856
- EP-A- 0 714 968
- WO-A-97/06779
- CH-A- 470 385
- FR-A- 2 741 799
- MARK H.F. ET AL: "Encyclopedia of Polymer Science and Engineering", 1990, JOHN WILEY & SONS , NEW YORK

## Description

La présente invention concerne l'utilisation de mélatonine ou de ses analogues pour la stabilisation des compositions comprenant des polymères gélifiants hydrophiles, en particulier dans des compositions cosmétiques ou pharmaceutiques topiques.

Les agents gélifiants hydrophiles sont d'usage très répandu dans les compositions cosmétiques ou pharmaceutiques topiques. Il s'agit notamment d'agents gélifiants tels que des polymères d'origine naturelle et/ou synthétique, parmi lesquels on peut citer les polymères carboxyvinyliques (carbomer), les copolymères acryliques tels que les copolymères d'acrylates/alkylacrylates, les polyacrylamides ou les polysaccharides éventuellement modifiés comme l'hydroxypropylcellulose.

Il est connu que ces polymères gélifiants hydrophiles perdent de leur viscosité sous l'effet de la lumière, plus particulièrement sous l'effet des rayonnements U.V. et pour résoudre ce problème, on ajoute généralement des filtres U.V.A et/ou U.V.B aux préparations les contenant. Toutefois, de tels filtres sont généralement hydrophobes et sont souvent incriminés dans des phénomènes d'intolérance tels que photo-allergie et/ou photo-toxicité, ce qui limite fortement leur utilisation dans des compositions cosmétiques ou pharmaceutiques topiques.

La demanderesse a trouvé de manière surprenante que la mélatonine ou ses analogues permettait de stabiliser les polymères gélifiants hydrophiles, en prévenant leur perte de viscosité sous l'action de la lumière.

La mélatonine, ou N-acétyl-5-méthoxytriptamine, sécrétée par la glande pinéale, est surtout connue pour son activité circadienne régulant la production d'hormones, en particulier pour son influence sur le rythme du sommeil.

Elle a également été décrite pour son activité antioxydante et son utilisation en dermo-cosmétique pour améliorer l'apparence de la peau (JP-A-61-221104; US-A-4,746,674), ou pour protéger la peau contre l'effet d'une irradiation aux rayonnements U.V. (EP-A-0438 856; E. Bangha & al., Dermatology 191, [2], 176, 1995).

Différentes compositions topiques comprenant de la mélatonine pour un usage thérapeutique ou cosmétique ont été décrites, comme des lotions de toilettes, des crèmes ou des laits de toilette (JP-A-61-221104). L'article de Bangha & al. concernant l'efficacité de la mélatonine pour supprimer l'érythème induit par les U.V.B décrit également l'utilisation de la mélatonine dans un gel de nanocolloïdes. La mélatonine est toujours employée comme agent actif, aucun document ne décrivant ou suggérant son influence sur la stabilité des polymères hydrophiles gélifiants. Il est également notable que la mélatonine est employée dans ces compositions essentiellement pour ses propriétés antioxydantes, en particulier pour la capture des radicaux libres, alors qu'elle n'est pas une molécule filtrante dans le spectre solaire, entre 280 et 800 nm.

La présente invention concerne donc l'utilisation de mélatonine ou d'au moins un de ses analogues pour stabiliser les compositions comprenant au moins un polymère gélifiant hydrophile. Par "stabiliser", on entend essentiellement prévenir leur perte de viscosité sous l'action de la lumière.

Parmi les analogues de la mélatonine, on peut notamment citer ses dérivés, tels que la 5-méthoxytryptamine, le 5-méthoxytryptophane, le 5-méthoxytryptophol, l'acide 5-méthoxyindole-3-acétique et la 6-hydroxymélatonine. On peut également citer des agonistes mélatoninergiques, tels que ceux décrits dans les demandes de brevets WO-A-95/17405, EP-A-0447285, EP-A-0527687, EP-A-0530087 et EP-A-0591057. Ces composés peuvent être d'origine naturelle ou synthétique.

D'une manière préférentielle, on emploiera la mélatonine.

Parmi les polymères gélifiants hydrophiles, on entend avantageusement les polymères d'origine naturelle et/ou synthétique, parmi lesquels on peut citer les polymères carboxyvinyliques (carbomer), les copolymères acryliques tels que les copolymères d'acrylates/alkylacrylates, les polyacrylamides ou les polysaccharides, comme la gomme de guar, de xanthane ou les celluloses, éventuellement modifiés comme l'hydroxypropylcellulose. Il peut s'agir aussi d'un mélange de ces polymères.

D'une manière préférentielle, le polymère gélifiant hydrophile est un polymère carboxyvinylique, par exemple commercialisé sous la marque Carbopol® par la société GOODRICH.

Conformément à la présente invention, l'utilisation de mélatonine ou de ses analogues permet de stabiliser des compositions cosmétiques ou pharmaceutiques, plus particulièrement topiques comprenant comme agent gélifiant un polymère gélifiant hydrophile. Les compositions topiques doivent contenir un milieu topiquement acceptable, c'est-à-dire compatible avec la peau, les muqueuses, les ongles, le cuir chevelu et les cheveux.

La présente invention concerne également un procédé de stabilisation d'une composition comprenant au moins un polymère gélifiant hydrophile, consistant à ajouter une quantité approprié de mélatonine ou d'au moins un de ses analogues pour prévenir la perte de viscosité dudit polymère sous l'action de la lumière.

D'une manière avantageuse, le rapport pondéral de la mélatonine ou ses analogues au polymère gélifiant hydrophile est compris entre 1/2000 et 20/1, de préférence compris entre 1/500 et1/2.

La présente invention concerne enfin les compositions cosmétiques ou pharmaceutiques, en particulier topiques, comprenant au moins un polymère gélifiant hydrophile et à titre d'agent de stabilisation dudit polymère gélifiant hydrophile, la mélatonine ou au moins un de ses analogues.

Ces compositions, comprennent entre 0,001% et 0,2% en poids de mélatonine ou ses analogues et entre 0,01% et 3% en poids de polymère gélifiant hydrophile. Le pourcentage en poids s'exprime par rapport au poids total de la composition.

Les compositions dans lesquelles se trouvent la mélatonine ou ses analogues peuvent se présenter sous toutes les formes galéniques à usage topique normalement utilisées comprenant au moins un agent gélifiant hydrophile, sous la forme notamment de solution aqueuse, d'émulsions de consistance liquide ou semi-liquide du type lait, ou de suspensions ou émulsions de consistance molle du type crème ou gel aqueux. Ces compositions sont préparées selon les méthodes usuelles de la technique.

Les quantités des différents constituants usuels des compositions sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes solaires), des fonds de teint fluides, des laits de démaquillage, des laits corporels de protection ou de soin, des laits solaires, des lotions, gels ou mousses pour le soin de la peau, comme des lotions de nettoyage, des lotions solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

De façon connue, la composition cosmétique ou dermatologique peut contenir également des adjuvants habituels dans le domaine cosmétique ou dermatologique, tels que les conservateurs, les antioxydants, les solvants, les parfums, les charges, les filtres, les absorbeurs d'odeur et les matières colorantes. Les quantités de ces différents adjuvants sont celles classiquement utilisées dans ces domaines, et par exemple de 0,01 % à 10 % du poids total de la composition.

La composition peut contenir des actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides, ou des actifs destinés notamment à la prévention et/ou au traitement des affections cutanées comme les agents modulant la différenciation et/ou la prolifération et/ou la pigmentation cutanée, les antibactériens, les antiparasitaires , les antifongiques, les agents anti-inflammatoires stéroïdiens ou non-stéroïdiens, les agents antiprurigineux, les agents anti-radicaux libres, les antiséborrhéiques tels que la progestérone, les antagonistes de CGRP ou les antagonistes de substance P ou encore les antagonistes de la bradykinine, et les agents antipelliculaires.

Les exemples ci-après illustrent la présente invention sans toutefois en limiter la portée. Dans ces exemples, les pourcentages sont exprimés en poids par rapport au poids total des compositions.

| **Exemple 1** | |
|---|---|
| Carbomer commercialisé sous la dénomination Carbopol 940 par la société GOODRICH | 0,50 % |
| Triéthanolamine | 0,50 % |
| Mélatonine | 0,18 % |
| Conservateurs | qs |
| Colorants | qs |
| Eau | qsp 100 % |

| **Exemple 2** | |
|---|---|
| Stéarate de glycérol | 0,50 % |
| PEG 100 stéarate | 2,00 % |
| Alcool cétylique | 0,40 % |
| Cyclométhicone | 3,00 % |
| Huile de soja | 3,00 % |
| Acrylates/C10-C30 alkyl acrylate crosspolymère (CTFA) commercialisé sous la dénomination Carbopol 1382 par la société GOODRICH | 0,30 % |
| Mélatonine | 0,05 % |
| Conservateurs | qs |
| Eau | qsp 100 % |

| **Exemple 3** | |
|---|---|
| Sodium laureth sulfate | 2,00 % |
| Glycérine | 3,00 % |
| Carbomer commercialisé sous la dénomination Carbopol 940 par la société GOODRICH | 0,20 % |
| Carraghenane | 0,30 % |
| Hydroxyde de sodium | 0,10 % |
| Mélatonine | 0,03 % |
| Conservateurs | qs |
| Eau | qsp 100 % |

### Test de stabilité

La composition de l'exemple 1 comprenant un polymère gélifiant hydrophile et de la mélatonine a été soumise à une exposition aux U.V. (longueurs d'onde comprises entre 300 et 830 nm) dans une enceinte fermée SUNTEST CPS-HERAEUS, les échantillons étant conditionnés dans des flacons de verre nu.

La viscosité de la composition 1 a été mesurée avant exposition, après 4 heures d'exposition et après 19 heures d'exposition et comparée à celle de la même composition ne comprenant pas de mélatonine (Témoin). Les mesures ont été réalisées au RHEOMAT 108 E/R à température constante (25°C) et la lecture a été faite après 10 minutes de cisaillement au mobile 3 et au mobile 2.

Les résultats obtenus sont reportés sur le tableau ci-après.

| Viscosité | Témoin | Composition de l'exemple 1 |
|---|---|---|
| Avant exposition U.V. | 37,5 Poises (M3) | 40 Poises (M3) |
| Après 4 heures d'exposition | 14 Poises (M3) (Diminution de viscosité 62 %) | 28 Poises (M3) (Diminution de viscosité 30 %) |
| Après 19 heures d'exposition | 2,5 Poises (M2) (Diminution de viscosité 93 %) | 16 Poises (M3) (Diminution de viscosité 60 %) |

Les résultats ci-dessus montrent que la mélatonine stabilise les polymères gélifiants puisqu'on observe une diminution de 60 % de la viscosité après 19 heures d'exposition pour la composition 1 selon l'invention, contre seulement 4 heures d'exposition pour le témoin sans mélatonine.

## Revendications

1. Utilisation de la mélatonine ou d'au moins un de ses dérivés pour stabiliser une composition comprenant au moins un polymère gélifiant hydrophile, pour prévenir la perte de viscosité dudit polymère sous l'action de la lumière.

2. Utilisation selon la revendication 1, **caractérisée en ce que** le dérivé de la mélatonine est choisi parmi la 5-méthoxytryptamine, 5-méthoxytryptophane, 5-méthoxytryptophol, l'acide 5-méthoxyindole-3-acétique, la 6-hydroxymélatonine et leurs mélanges.

3. Utilisation selon la revendication 1, **caractérisée en ce que** l'on utilise la mélatonine.

4. Utilisation selon l'une des revendications 1 à 3, **caractérisée en ce que** le polymère gélifiant hydrophile est choisi parmi les polymères carboxyvinyliques (carbomer), les copolymères acryliques, les polyacrylamides, les polysaccharides éventuellement modifiés et leurs mélanges.

5. Utilisation selon la revendication 4, **caractérisée en ce que** le polymère gélifiant hydrophile est un polymère carboxyvinylique.

6. Utilisation selon l'une des revendications 1 à 5, **caractérisée en ce que** le rapport pondéral de la mélatonine ou de son dérivé au polymère gélifiant hydrophile est compris entre 1/2000 et 20/1, de préférence compris entre 1/500 et 1/2.

7. Utilisation selon l'une des revendications 1 à 6, pour stabiliser une composition cosmétique ou pharmaceutique.

8. Procédé de stabilisation d'une composition comprenant au moins un polymère gélifiant hydrophile, consistant à ajouter une quantité comprise entre 0,001% et 0,2% en poids de mélatonine ou d'au moins un de ses dérivés par rapport au poids total de la composition, pour prévenir la perte de viscosité dudit polymère sous l'action de la lumière.

9. Composition cosmétique ou pharmaceutique topique comprenant au moins un polymère gélifiant hydrophile, **caractérisée en ce qu'**elle comprend, à titre d'agent de stabilisation dudit polymère gélifiant hydrophile, de la mélatonine ou au moins un de ses dérivés en une quantité comprise entre 0,001% et 0,2% en poids par rapport au poids total de la composition.

10. Composition selon la revendication 9, **caractérisée en ce qu'**elle comprend entre 0,01% et 3% en poids de polymère gélifiant hydrophile, par rapport au poids total de la composition.

## Patentansprüche

1. Verwendung von Melatonin oder mindestens eines seiner Derivate zur Stabilisierung einer Zusammensetzung, die mindestens ein hydrophiles gelierendes Polymer enthält, um dem Viskositätsverlust des Polymers unter Lichteinwirkung vorzubeugen.

2. Verwendung nach Anspruch 1, **dadurch gekennzeichnet, dass** das Melatoninderivät unter 5-Methoxytryptamin, 5-Methoxytryptophan, 5-Methoxytryptophol, 5-Methoxy-3-indolessigsäure, 6-Hydroxymelatonin und den Gemischen dieser Verbindungen ausgewählt ist.

3. Verwendung nach Anspruch 1, **dadurch gekennzeichnet, dass** Melatonin verwendet wird.

4. Verwendung nach einem der Ansprüche 1 bis 3, **dadurch gekennzeichnet, dass** das hydrophile gelierende Polymer unter Carboxyvinylpolymeren (Carbomer), Acrylcopolymeren, Polyacrylamiden, gegebenenfalls modifizierten Polysacchariden und den Gemischen dieser Verbindungen ausgewählt ist.

5. Verwendung nach Anspruch 4, **dadurch gekennzeichnet, dass** es sich bei dem hydrophilen gelierenden Polymer um ein Carboxyvinylpolymer handelt.

6. Verwendung nach einem der Ansprüche 1 bis 5, **dadurch gekennzeichnet, dass** das Gewichtsverhältnis von Melatonin oder seinem Derivat zu dem hydrophilen gelierenden Polymer im Bereich von 1/2 000 bis 20/1 und vorzugsweise von 1/500 bis 1/2 liegt.

7. Verwendung nach einem der Ansprüche 1 bis 6 zur Stabilisierung einer kosmetischen oder pharmazeutischen Zusammensetzung.

8. Verfahren zur Stabilisierung einer Zusammensetzung, die mindestens ein hydrophiles gelierendes Polymer enthält, das darin besteht, Melatonin oder mindestens eines seiner Derivate in einem Mengenanteil von 0,001 bis 0,2 Gew.-%, bezogen auf das Gesamtgewicht der Zusammensetzung, zuzugeben, um dem Viskositätsverlust des Polymers unter Lichteinwirkung vorzubeugen.

9. Topische kosmetische oder pharmazeutische Zusammensetzung, die mindestens ein hydrophiles gelierendes Polymer enthält, **dadurch gekennzeichnet, dass** sie als Stabilisator des hydrophilen gelierenden Polymers Melatonin oder mindestens eines seiner Derivate in einem Mengenanteil im Bereich von 0,001 bis 0,2 Gew.-%, bezogen auf das Gesamtgewicht der Zusammensetzung, enthält.

10. Zusammensetzung nach Anspruch 9, **dadurch gekennzeichnet, dass** sie 0,01 bis 3 Gew.-% des hydrophilen gelierenden Polymers, bezogen auf das Gesamtgewicht der Zusammensetzung, enthält.

## Claims

1. Use of melatonin or of at least one of the derivatives thereof for stabilizing a composition comprising at least one hydrophilic gelling polymer, for preventing the loss of viscosity of the said polymer under the action of light.

2. Use according to Claim 1, **characterized in that** the melatonin derivative is chosen from 5-methoxytryptamine, 5-methoxytryptophan, 5-methoxytryptophol, 5-methoxyindole-3-acetic acid, 6-hydroxymelatonin and mixtures thereof.

3. Use according to Claim 1, **characterized in that** melatonin is used.

4. Use according to one of Claims 1 to 3, **characterized in that** the hydrophilic gelling polymer is chosen from carboxyvinyl polymers (carbomer), acrylic copolymers, polyacrylamides, optionally modified polysaccharides and mixtures thereof.

5. Use according to Claim 4, **characterized in that** the hydrophilic gelling polymer is a carboxyvinyl polymer.

6. Use according to one of Claims 1 to 5, **characterized in that** the weight ratio of the melatonin or of the derivative thereof to the hydrophilic gelling polymer is between 1/2000 and 20/1, preferably between 1/500 and 1/2.

7. Use according to one of Claims 1 to 6, for stabilizing a cosmetic or pharmaceutical composition.

8. Process for stabilizing a composition comprising at least one hydrophilic gelling polymer, consisting in adding an amount of between 0.001% and 0.2% by weight of melatonin or of at least one of the derivatives thereof relative to the total weight of the composition for preventing the loss of viscosity of the said hydrophilic gelling polymer under the action of light.

9. Topical cosmetic or pharmaceutical composition comprising at least one hydrophilic gelling polymer, **characterized in that** it comprises melatonin or at least one of the derivatives thereof in an amount of between 0.001% and 0.2% by weight relative to the total weight of the composition, as an agent for stabilizing the said hydrophilic gelling polymer.

10. Composition according to Claim 9, **characterized in that** it comprises between 0.01% and 3% by weight of hydrophilic gelling polymer, relative to the total weight of the composition.
